# EUROPEAN PATENT APPLICATION

(11) **EP 1 563 757 A1**
(43) Date of publication of application: **17.08.2005**
(21) Application number: 04003392.0
(22) Date of filing: 16.02.2004
(51) Int. Cl.: A45D 19/02, A45D 24/24

(54) **Hair dyeing device**

(71) Applicant: Hayun, Yael, Ramat Gan (IL)
(72) Inventor: Hayun, Yael, Ramat Gan (IL)
(74) Representative: Becker Kurig Straus

(57) **Abstract**

A simple, safe and discrete method for dyeing graying hairs is disclosed, whereby a device similar to a marker is used to selectively apply dye to the hair. A device exceptionally useful in implementing the method of the present invention is also disclosed, the device substantially being substantially a marker having features on the application end giving the application end a comb-like cross section.

## Description

### FIELD AND BACKGROUND OF THE INVENTION

The present invention relates to the dyeing of hair and specifically to a device and a method for the dyeing of hair roots, individual or small groups of hairs anywhere on the body.

In some cultures graying hair is considered unattractive. As a result, graying hair is often concealed by dyeing. A convenient method to dye hair is to go to a hair-dressing professional who dyes the hair, an expensive and time-consuming process. An alternative is to purchase a hair-dyeing kit to dye one's own hair. Although cheaper, self-dyeing is even more time consuming, often gives inadequate results and is quite messy.

There are additional disadvantages to these two hair-dyeing methods.

First, since all the hair is immersed or coated in a dyeing solution, users of both methods are exposed to unpleasant and potentially hazardous chemical compounds.

Second, these methods adequately color all the hair of the head, but do not address the need for selective and localized hair dyeing. Selective and localized hair dyeing is needed to dye individual grey hairs, to dye grey roots, or to dye only a single lock or area of hair.

Third, these methods do not provide an adequate solution for dyeing hair in sensitive places such as near the eyes or the pubis. The proximity to sensitive areas makes it dangerous to dye hairs in such areas oneself, although even when a hair-care professional dyes these hairs there is an element of risk.

Fourth, these methods do not provide an adequate solution for dyeing hair in intimate places such as the pubis. Self-dyeing is dangerous and difficult, but many people feel uncomfortable having such a procedure performed by a stranger. As a result, often hair in these areas remains grey, to the distress and discomfort of the person thus afflicted.

Lastly, as these two dyeing methods are expensive, messy and time consuming, it is not possible to dye hair often or on short notice. This is a significant problem, for example, when an individual identifies a grey hair immediately before an important social occasion when there is not enough time or no convenient place to dye the hair. Also, due to the constant growth of the hair, one is presented with the choice of having gray roots or visiting a hair-care professional on a weekly basis.

A number of devices have been proposed to overcome some of these disadvantages.

European Patent Application EP 0 790 052 A1 teaches the application of a mascara-type color with a brush to the roots of gray hairs. Mascara is an encaustic comprising a carrier (for example, a wax) with pigment particles suspended therein and is typically applied to eyelashes. Thus mascara coats the outer surface of hairs to which it is applied. As a result, hair to which mascara is applied becomes thicker and longer. Mascara is designed to be removable so as to prevent adhesion of the upper and lower eyelids. Clearly, for both these reasons, mascara-type coloring is inappropriate for dyeing graying hair-roots.

U.S. 5,937,866 discloses a hair-dye applicator. For use, liquid dye is prepared and poured into a container. The lid of the container is an applicator head having comb teeth and pores. When the comb teeth are passed through hair, hair-dye flows through the pores, and by the action of the comb teeth is spread evenly over the hairs. In U.S. 5,024,243 a related device is disclosed having a complex valve system to control the amount of dye dispensed from a squeeze bottle. In U.S. 5,337,763 a related device having two chambers for storing two components of a single hair-dye is disclosed. Although dyeing hair using these devices is less messy than standard hair-dyeing methods, these devices are not suitable for dyeing individual hairs or for dyeing exclusively hair roots. Further, there is a good chance that hair-dye will drip from the comb teeth, staining the scalp or surroundings. The danger of dripping precludes the use of these devices for dyeing hair in the proximity of sensitive areas.

U.S. 2,299,296 discloses a hair operator's instrument that includes, in addition to a comb supplied with liquid dye through pores similar to that disclosed in U.S. 5,937,866, an absorbent applicator supplied with liquid dye through pores. The use of the absorbent applicator prevents dripping. However the size and shape of the applicator is not suitable for dyeing individual hairs or for dyeing exclusively hair roots. Further, a given applicator is for a single use: multiple dye components are mixed and poured into the device and a new applicator is attached. After one use, both excess dye and the used applicator are discarded.

U.S. 5,778,902 discloses a hair dyeing device in the form of a pen, the hair dyeing device having two chambers for storing two components of a hair-dye and an absorbent applicator tip. For use, the contents of the two chambers are mixed inside the device to make a useable dye. The use of a pen format allows for localized touching up of individual hairs and hair roots. The ease of accurate use makes this device suitable for the dyeing of hairs in sensitive and intimate areas. Despite this, the shape of the applicator tip of the device is suitable for dyeing no more than one hair at a time. The use of a two component dye and other design features makes the device unsuitable for repeated use after storage. The need for mixing the components reduces the simplicity of use of the device.

As is clear to one skilled in the art, the methods discussed above are unsuitable for the dyeing of individual hairs or limited areas of grey hairs. The methods discussed above are not suitable for dyeing hairs in intimate or sensitive areas of the body. Although the device of U.S. 5,778,902 can be used to color grey hairs, this is done only a single strand at a time.

There is a need for a device and a method of application of dye to hair that allows a person to dye specific sections of hair, quickly, cleanly and accurately. There is a need for device that is suitable for the coloring of grey hair roots.

### SUMMARY OF THE INVENTION

The present invention provides a method and a device to fill this need. The device of the present invention is a hair-dyeing device that is substantially a marker configured to apply a dye, preferably a single-component dye, to hair.

By marker is meant herein an applicator for applying dye to hair, the applicator configured to wick dye from a reservoir of some kind to an application end of the applicator. The advantage of using such an applicator is that dye does not drip from such an applicator during use. The technical details of markers (alternatively known in the art as soft-tip pens, fiber-tip pens and felt-tip pens) and the manufacture thereof are well known. Markers suitable for use according to the present invention are manufactured, for example, by such companies as Staedler Mars GmbH & Co. (Nürnberg, Germany), Faber-Castell Vertrieb GmbH (Stein, Germany), Dixon Ticonderoga Co. (Heathrow, Florida), Pentel Co. Ltd. (Tokyo, Japan) and Pilot Corporation (Tokyo, Japan).

According to the teachings of the present invention there is provided a method for the dyeing of hair, comprising a) providing a device having a wickable dye contained within a barrel and an applicator having an application end and a base end, configured to wick the dye from the base end towards the application end, wherein the base end of the applicator is in permanent contact with the dye and b) applying the dye to at least one hair on the body of a person by bringing the application end of the applicator in contact with the at least one hair.

Although a standard marker can be used in implementing the method of the present invention, it is preferable that a device of the present invention be used.

According to the teachings of the present invention, there is provided a device for the dyeing of hair, comprising: a) a wickable dye contained within a barrel; and b) an applicator having an application end and a base end, configured to wick the dye from the base end towards the application end wherein the application end has features, the features giving the application end a comb-like cross-section.

According to a feature of the device present invention, the base end of the applicator is in permanent contact with the dye.

According to a feature of the device of the present invention, the features giving the application end a comb-like cross-section are configured to wick the dye, preferably being an integral part of the applicator.

According to a further feature of the device of the present invention, at least part of the features are configured to not wick the dye, that part being the part of the features corresponding to the tips of the teeth of the comb-like cross section.

According to a further feature of the present invention, the dye is a single component dye. According to a feature of the present invention, the dye includes a coloring material, the coloring material being substantially insoluble in water.

According to a further feature of the present invention, the dye is stored inside no more than one chamber of the barrel of the device used to implement the method of the present invention.

According to a feature of the present invention, a dye stored in the barrel of a device used in implementing the method of the present invention is stored as a liquid or as a liquid absorbed in an absorbent material.

According to a feature of the present invention, the application end of a device used in implementing the method of the present invention has features, the features giving the application end a comb-like cross-section.

According to a still further feature of the present invention, these features are configured to wick the dye and are preferably an integral part of the applicator. According to a still further feature of the present invention, at least part of these features (preferably the part if the features that corresponds to the tips of the teeth of the comb-like cross section) is configured to not wick the dye.

The features that give the application end a comb-like cross section can be a separate piece or an integral part of the application end. The features can be of any shape that in cross section is comb-like. For example, included is that the application end is spiral: in cross section a spiral is comb-like.

By dye is meant herein any coloring composition such as a dye, ink, color, pigment, paint, tint, colorant or stain that can be wicked to the application end of a device used to implement the method of the present invention and therefrom applied to hair. Such coloring compositions generally include a coloring material and a carrier or solvent. The mechanism by which the dye imparts color to hair is not salient to the teachings of the present invention. It is preferred that the dye be permanent, meaning that subsequent to application the dye imparts a color to hair for a significant amount of time. It is preferred that the dye not wash away in sweat, rain or a shower, does not come off (*i.e.* to the point of staining hats, articles of clothing, hands) even when heated (*e.g*. by body heat, the sun, photography lamps). Permanent dyes according to the present invention include what are referred to in the art of hair coloring as permanent colors, semi-permanent colors, demi-permanent colors and color washes (temporary colors removed by washing with shampoo).

By single-component dye is meant a dye that is ordinarily provided and is storable in a single container as a single ready-to-use mixture or solution. The meaning of the term "single-component dye" as used herein includes dyes that settle into two or more distinct chemical entities even when stored in the same vessel but are mixed, for example by agitation of the vessel, before use.

It is important to note that in the art, non-single component dyes are ordinarily used for the dyeing of hair, *e.g.* as used in U.S. 5,778,902. Usually two-component dyes (comprising an alkaline-containing component and an oxidizing-agent containing component) are mixed immediately before use. Upon mixing the components begin to react and as a result, the mixed dye quickly becomes ineffective and cannot be stored.

Many dyes can be used in implementing the present invention. Since the present invention avoids contact of the dye with the scalp, does not drip and only small amounts of dye are used, dyes that are otherwise considered unsuitable for hair coloring can be used. Such dyes include dyes normally associated with permanent markers and include coloring compositions having as components non-aqueous solvents and coloring materials that are partially, barely or completely insoluble in water. Some of these coloring materials and some of the solvents are considered dangerous. Dangerous solvents used in permanent markers include xylene (in 8700 series Dixon RediMark Marker, Dixon Ticonderoga Corporation and in N50 permanent markers of Pentel Co. Ltd.), diacetone alcohol (in Super Sharpie Faber Castell GmbH) and benzyl alcohol (in MS50A Permanent Markers of Pentel Co. Ltd).

### BRIEF DESCRIPTION OF THE DRAWINGS

The invention is herein described, by way of example only, with reference to the accompanying drawings, where:
FIG. 1A is a cut-out view of a marker useful in implementing the present invention where dye is stored as a liquid;
FIG. 1B is a cut-out view of a marker useful in implementing the present invention wherein dye is stored in a reservoir made of an absorbent material;
FIGS. 2A-2C are schematic depictions of application ends of devices of the present invention;
FIG. 3 is a view showing a device of the present invention used in coloring hair in sensitive and intimate areas of the body; and
FIGS. 4A-4B are views showing devices of the present invention used in coloring selected areas of scalp hair.

### DETAILED DESCRIPTION OF EMBODIMENTS OF THE INVENTION

The principles and use of the method and device according to the present invention may be better understood with reference to the drawings and the accompanying description. In the drawings, like reference numerals designate equivalent parts

The method of the present invention can be considered to be the use of a marker configured for dyeing hair. Although any hair can be dyed, the unique features of the invention allow safe and accurate dyeing of pubic hair, mustache, beards, sideburns, eyebrows and areas of hair on the head such as roots, groups or individual hairs.

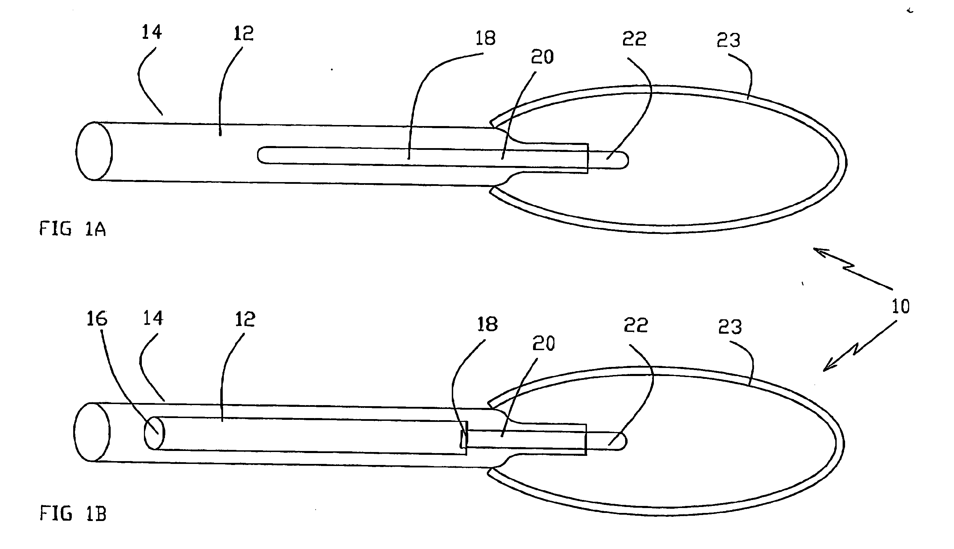

In Figure 1 are depicted two markers **10** useful in implementing the method of the present invention, in a cut-out view. A dye **12** is stored in a barrel **14.** In Figure 1A, barrel **14** is a reservoir for dye **12** stored as a liquid. In Figure 1B, a reservoir **16** for dye **12** is held within barrel **14**, reservoir **16** being substantially a cylinder of absorbent material. In continuous contact with dye **12** is a base end **18** of an applicator **20.** Applicator **20** is made of a material such as felt that wicks dye **12** towards an application end **22** of applicator **20**. To prevent evaporation of the solvent in dye **12**, devices **10** are fitted with a cap **23**.

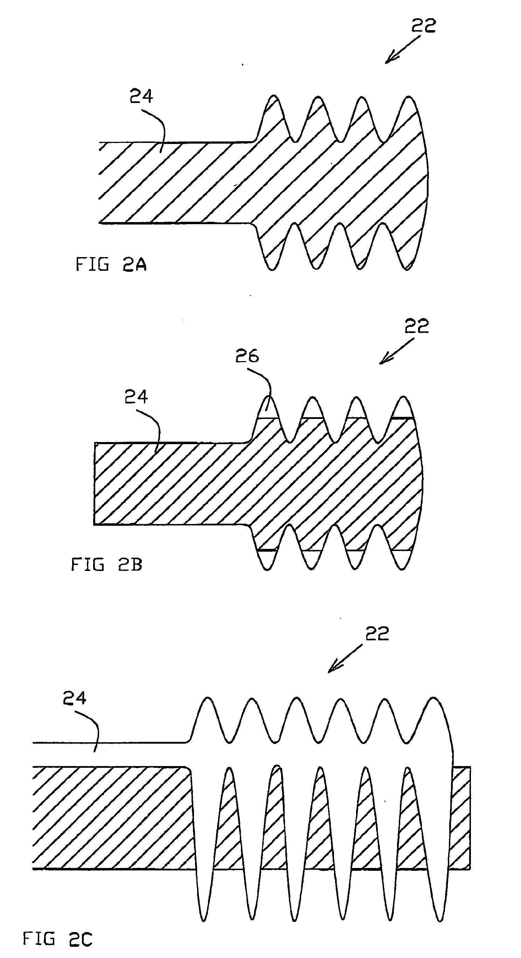

The use of markers **10** such as depicted in Figures 1 in implementing the method of the present invention may be considered less then perfect as it is difficult to color a plurality of hairs or hair roots due to the small size and inconvenient shape of application end **22.** These problems are overcome by the device of the present invention. In general, a device of the present invention is a substantially a marker **10** as depicted in Figures 1, where the application end **22** of applicator **20** has features that give application end **22** a comb-like cross section. In Figures 2 are depicted embodiments of application ends **22** of devices of the present invention, all having a comb-like cross section.

In Figure 2A, the comb-like cross section of application end **22** is a result of the shape of application end **22.** The "comb-teeth" **24** of the cross section are the result of the fact that application end **22** is spiral. Thus "comb-teeth" **24** are an integral part of application end **22** and are made of the same material as application end **22**. During use, dye **12** is wicked into application end **22** also through "comb-teeth" **24**. The comb-like cross section of application end **22** allows hair to enter the space between "comb-teeth" **24** and to be colored from more than one side. Thus, a plurality of hairs can be simultaneously, completely and quickly dyed.

Application end **22** depicted in Figure 2B is very similar to application end **22** depicted in Figure 2A excepting that part of the features that give application end **22** a comb-like cross section are designed not to wick dye **12.** Specifically, to the part of application end **22** that corresponds to the tips of "comb-teeth" **24** is attached impermeable metal foil **26**. Metal foil 26 prevents any transfer of dye **12** if application end **22** inadvertently makes incidental contact with the scalp or other part of the body.

In Figure 2C, the comb-like cross section of application end **22** is a result of the attachment of a comb-tip **28** to encircle a cylindrical end **30** of application end **22**. Comb-tip **28** is a plurality of substantially circular plastic disks arrayed substantially in parallel to each other. Comb-tip **28** does not wick dye and simply acts to separate and guide hairs to be dyed to proximity with cylindrical end **30** to be dyed and thereby to protect the scalp or other body parts from dye transfer.

Application end **22** depicted in Figure 2A is cheap, easy to manufacture and ideal for the dyeing of a single or a plurality of graying hairs. A plurality of hairs is lifted upwards between "comb-teeth" **24** and is quickly and efficiently colored. Minor dyeing of the scalp will not be noted.

Application ends **22** as depicted in Figure 2B or Figure 2C are suitable for dyeing sensitive areas such as the pubis, eyebrows, mustache or areas where no dyeing of the surrounding areas can be countenanced.

An advantage of an application end **22** depicted in Figure 2C is that comb-tip **28** (or equivalent) can be easily designed to be reversibly attachable to cylindrical end 30. Thus, a plurality of comb-tips **28** with varying pitches and depths can be provided for use with a device of the present invention. A comb-tip **28** having shallow and narrow spaces between comb teeth is used to dye the hair of eyebrows or graying roots, whereas a comb-tip 28 having deeper and wider spaces is used for dyeing the hairs of the pubis or sideburns.

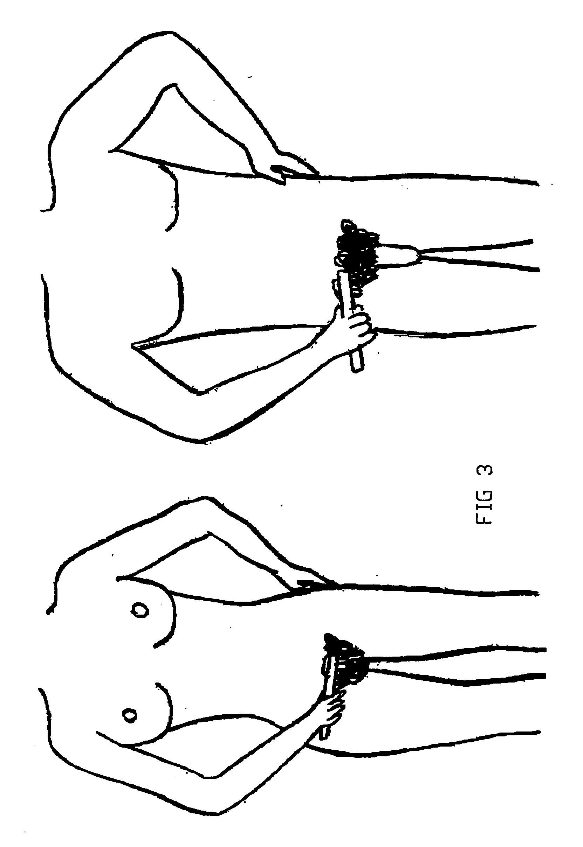

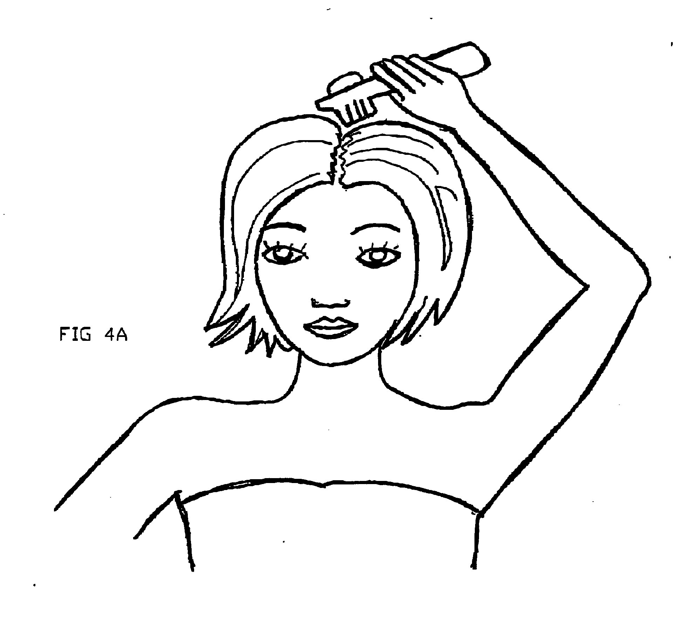

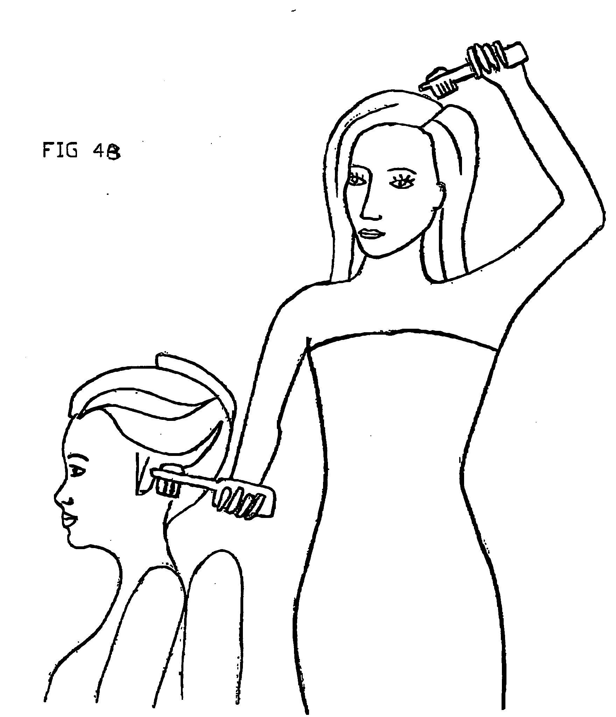

It is clear to one skilled in the art how to implement the method of the present invention, especially using a device of the present invention as depicted in Figures 2. Cap **23** is removed, exposing application end **22**. While looking in a mirror, a user carefully and selectively colors only graying roots and graying hairs, without coloring the scalp. Since the device of the present invention does not leak, the device can be used to dye hairs on any part of the body. The method of the present invention is depicted in Figures 3 and 4. In Figure 3 the method of the present invention is depicted for dyeing pubic hair. In Figures 4 is depicted how a person dyes graying roots of dyed hair for themselves (Figure 4A and 4B) or for another person (Figure 4B).

A device used in implementing the the present invention can easily be kept in proximity of a user, for example in a hand bag or glove-compartment of a motor vehicle. As is well-known to one skilled in the art, such a device does not leak, can be stored for extended periods of time and can safely be transported. The device can be used almost anywhere even in the restroom of a restaurant or inside of a motor vehicle. Thus quick and discrete use is possible. Further the ease of use allows absolutely safe and accurate application to sensitive and discrete parts of the body.

It is important to note that the primary purpose for which the present invention has been made is for the dyeing of graying hairs to match non-graying hairs. It is clear to one skilled in the art that with appropriate dyes, different effects such as frosting, streaking highlighting or other types of decoration can be achieved using the method and device of the present invention.

It will be appreciated that the above examples and descriptions are intended only to serve as examples and that many other embodiments are possible within the spirit and the scope of the present invention.

## Claims

1. A device for the dyeing of hair, comprising:
a. a wickable dye contained within a barrel; and
b. an applicator having an application end and a base end, configured to wick said dye from said base end towards said application end
wherein said application end has features, said features giving said application end a comb-like cross-section.

2. The device of claim 1 wherein said base end of said applicator is in permanent contact with said dye.

3. The device of claim 1 wherein said features are configured to wick said dye.

4. The device of claim 1 wherein said features are an integral part of said applicator.

5. The device of claim 1 wherein at least part of said features is configured to not wick said dye.

6. The device of claim 5 wherein a part of said features corresponding to tips of teeth of said comb-like cross section are configured to not wick said dye.

7. A method for the dyeing of hair, comprising:
a. providing a device having
i. a wickable dye contained within a barrel; and
ii. an applicator having an application end and a base end, configured to wick said dye from said base end towards said application end, wherein said base end of said applicator is in permanent contact with said dye and
b. applying said dye to at least one hair on the body of a person by bringing said application end in contact with said at least one hair.

8. The method of claim 7 wherein said dye is contained within said barrel as a liquid.

9. The method of claim 7 wherein said dye is contained within said barrel absorbed in an absorbent material.

10. The method of claim 7 wherein said dye is a single component dye.

11. The method of claim 7 wherein said dye is stored inside no more than one chamber of said barrel.

12. The method of claim 7 wherein said dye includes a coloring material, said coloring material being substantially insoluble in water.

13. The method of claim 7 wherein said application end has features, said features giving said application end a comb-like cross-section.

14. The device of claim 13 wherein said features are configured to wick said dye.

15. The device of claim 13 wherein said features are an integral part of said applicator.

16. The device of claim 13 wherein at least part of said features is configured to not wick said dye.

17. The device of claim 16 wherein a part of said features corresponding to tips of teeth of said comb-like cross section are configured to not wick said dye.
